# EUROPEAN PATENT APPLICATION

(11) **EP 2 087 860 A1**
(43) Date of publication of application: **12.08.2009**
(21) Application number: 09250301.0
(22) Date of filing: 06.02.2009
(51) Int. Cl.: A61F 5/00

(54) **Powering implantable restriction systems using temperature**

(30) Priority: 07.02.2008 US 27817
(71) Applicant: Ethicon Endo-Surgery, Inc., Cincinnati, OH 45242 (US)
(72) Inventor: Dlugos Jr. , Daniel F., Middletown, Ohio 45044 (US); Ortiz, Mark S., Milford, Ohio 45150 (US); Marcotte, Amy L., Mason, Ohio 45040 (US); Doll, Kevin R., Mason, Ohio 45040 (US); Plescia, David N., Cincinnati, Ohio 45227 (US); Stokes, Michael J., Cincinnati, Ohio 45233 (US)
(74) Representative: Tunstall, Christopher Stephen

(57) **Abstract**

Various powering devices are provided for transferring and/or generating energy from numerous sources to a communicating member implanted in a patient. The energy transferred to or generated by the communicating member can be used to provide power to an implantable restriction system configured to form a restriction in a pathway.

## Description

### FIELD OF THE INVENTION

The present invention relates to methods and devices for providing power to implantable restriction systems.

### BACKGROUND OF THE INVENTION

Obesity is becoming a growing concern, particularly in the United States, as the number of obese people continues to increase, and more is learned about the negative health effects of obesity. Morbid obesity, in which a person is 100 pounds or more over ideal body weight, in particular poses significant risks for severe health problems. Accordingly, a great deal of attention is being focused on treating obese patients. One method of treating morbid obesity has been to place a restriction device, such as an elongated band, about the upper portion of the stomach. Gastric bands have typically comprised a fluid-filled elastomeric balloon with fixed endpoints that encircles the stomach just inferior to the esophageal-gastric junction to form a small gastric pouch above the band and a reduced stoma opening in the stomach. When fluid is infused into the balloon, the band expands against the stomach creating a food intake restriction or stoma in the stomach. To decrease this restriction, fluid is removed from the band. The effect of the band is to reduce the available stomach volume and thus the amount of food that can be consumed before becoming "full."

Food restriction devices have also comprised mechanically adjusted bands that similarly encircle the upper portion of the stomach. These bands include any number of resilient materials or gearing devices, as well as drive members, for adjusting the bands. Additionally, gastric bands have been developed that include both hydraulic and mechanical drive elements. It is also known to restrict the available food volume in the stomach cavity by implanting an inflatable elastomeric balloon within the stomach cavity itself. The balloon is filled with a fluid to expand against the stomach walls and, thereby, decrease the available food volume within the stomach.

With each of the above-described food restriction devices, safe, effective treatment requires that the device be regularly monitored and adjusted to vary the degree of restriction applied to the stomach. Traditionally, adjusting a gastric band required a scheduled clinician visit during which a Huber needle and syringe were used to penetrate the patient's skin and add or remove fluid from the balloon via the injection port. More recently, implantable pumps have been developed which enable non-invasive adjustments of the band. An external programmer communicates with the implanted pump using telemetry to control the pump. During a scheduled visit, a physician places a hand-held portion of the programmer near the gastric implant and transmits command signals to the implant. The implant in turn adjusts the band and transmits a response command to the programmer.

Implants such as those described above include electronics which require a power source that is sufficient for the intended function, such as making adjustments to the gastric band. Such devices may be internally powered by a battery or capacitor while others may be powered by an externally coupled power source or passive telemetry system. When coupling externally, the efficiencies between the implant and external device diminish substantially as the distance between them increases. There can also be significant power losses through tissue.

Accordingly, there is a need for methods and devices for charging implanted electronics efficiently through tissue by using external and/or non-invasive techniques. It would also be advantageous for a patient to be able to recharge implants without having to travel to a scheduled clinician visit.

### SUMMARY OF THE INVENTION

The present invention provides methods and devices for providing power to an implantable restriction system. In one exemplary embodiment, a system for forming a restriction in a patient is provided and includes an implantable restriction device adapted to form a restriction in a pathway within a patient. For example, the implantable restriction device can include a gastric band and a housing in communication with the gastric band. The implantable restriction device can also include a communicating member that powers the implantable restriction device. The system can further include an external apparatus that is operable to communicate with the communicating member by sending power and/or data signals to the communicating member and/or by receiving data signals from the communicating member. The communicating member can also be configured to send data signals to an external device. The external apparatus can optionally include a gauge that is effective to indicate whether the external apparatus is effectively communicating with the communicating member.

In one embodiment, the communicating member can be adapted to convert light waves into energy, and the external apparatus can be an energy transfer apparatus having a light source that is operable to communicate light to the communicating member. The energy transfer apparatus can include a gauge effective to indicate whether the light waves are being communicated between the light source and the communicating member effective to power the implantable restriction device or communicate data. In an embodiment, the communicating member can be a photovoltaic cell array, a silicon nanowire bundle, or a crystalline silicone cell array, and the light source can emit infrared light waves in a range of about 0.70 µm to 1,000 µm. Alternatively, the light source can emit visible light waves in a range of about 400 nm to 1,000 nm or ultraviolet light waves in a range of about 280 nm to 400 nm.

In another embodiment, the communicating member can be adapted to utilize a temperature differential to power the implantable restriction device, the energy transfer apparatus can have a temperature source operable to create a temperature differential across the communicating member to power the implantable restriction device. In an exemplary embodiment, the communicating member is a thermogenerator. The temperature source can be, for example, ice, a thermoelectric cooler, a heating source, and a blood vessel. The communicating member can be configured to utilize a temperature differential between the temperature source and an anatomical reference temperature to produce energy to power the implantable restriction device. In another embodiment, the gauge can be effective to indicate whether a temperature differential exists between the temperature source and the communicating member effective to power the implantable restriction device.

In another embodiment, the communicating member can have a kinetic motion apparatus operable to convert motion into energy to power the implantable restriction device. The kinetic motion apparatus can include a housing, a magnet disposed within the housing, and a wire coil disposed around the housing. The wire coil can be in electrical communication with the implantable restriction device and the magnet can be configured to move relative to the wire coil to create electrical energy to power the implantable restriction device. The kinetic motion apparatus can further include a storage device for storing the electrical energy produced from movement of the magnet. The system may also include an external device that may include a driver adapted to produce corresponding oscillations, vibrations, or other motions in the kinetic motion apparatus effective to power the implantable restriction device. Alternatively, an external oscillating electromagnet can induce sympathetic oscillations in the magnet disposed within the housing. In another embodiment, the gauge can be adapted to indicate a charge status of the implantable restriction device.

In a further exemplary embodiment, a kinetic motion apparatus can include a counterweight coupled to a drive gear and configured to rotate freely about a pivot point when the kinetic motion apparatus is rotated in response to patient movement. The kinetic motion apparatus can also include an electric generator configured to receive mechanical energy from the drive gear and convert it to electrical energy to power the implantable restriction device.

In one embodiment, a kinetic motion apparatus can include a piezoelectric device configured to convert internal muscle and/or organ movement within a patient into electrical energy to power the implantable restriction device. The piezoelectric device can also be configured to convert digestive movement of a patient's stomach against the gastric band into electrical energy to power the implantable restriction device.

Methods are also provided for powering an implantable restriction device. In one embodiment, the method can include activating a light source to transfer light through tissue to a communicating member disposed within an implantable restriction device. The communicating member can convert the light to electrical current to power the implantable restriction device. The light source can be on an external device, and the method can further include positioning the external device adjacent to a skin surface and in proximity to the communicating member implanted within tissue. The external device can also optionally include a gauge that indicates whether light transferred between the light source and the communicating member is effective to power the implantable restriction device. Additionally, the external device can receive data from the communicating member which includes at least one measurement of pressure of fluid within the implantable restriction device. In an exemplary embodiment, the light source emits infrared light with a wavelength in a range of about 0.70 µm to 1,000 µm. Alternatively, the light source emits visible light with a wavelength in a range of about 400 nm to 750 nm or ultraviolet light with a wavelength in the range of about 280 nm to 400 nm.

In another embodiment, a method is provided for powering an implantable restriction device and includes placing a temperature source on a tissue surface adjacent to a communicating member disposed within an implantable restriction device implanted in a patient. The communicating member utilizes a temperature differential to power the implantable restriction device. The communicating member may be placed close to the skin such that it resides in a temperature gradient between the external environment and the body core. Alternatively, the thermogenerator can be placed in contact with a large blood vessel since the body uses the blood stream to convey heat to and from the body. Thus, a natural temperature gradient exists in the body with may be used to generate power. The temperature source can be on an external device, and the external device can receive data from the communicating member. The external device can also include a gauge that indicates whether a temperature differential exists between the temperature source and the communicating member effective to power the implantable restriction device. The data can include at least one measurement of pressure of fluid within the implantable restriction device. In one embodiment, the temperature source can be ice, a thermoelectric cooler, and/or a heating source placed on or near a tissue surface adjacent to the thermogenerator creating a temperature differential with an anatomical reference temperature across the thermogenerator to produce electrical current to power the implantable restriction device.

In still another embodiment, a method for providing power to an implantable restriction device is provided and includes driving a communicating member coupled to an implantable restriction device implanted in a patient to power the implantable restriction device, where the communicating member includes a kinetic motion apparatus. The kinetic motion apparatus can include a metal wire and a magnet and the metal wire and a magnetic field created by the magnet move relative to one another, thereby generating electrical energy to power the implantable restriction device. In an exemplary embodiment, the metal wire and the magnetic field move relative to one another in response to motion by the patient. The kinetic motion apparatus can also be driven by an external oscillating electromagnet that induces sympathetic oscillations in the magnet. Alternatively, the kinetic motion apparatus is driven by a vibration element that causes the metal wire to move through the magnetic field. In another embodiment, the communicating member can be in communication with an external device that receives data from the communicating member and which can include a gauge that indicates a charge status of the implantable restriction device. The kinetic motion apparatus can alternatively include a counterweight coupled to a drive gear that rotates freely about a pivot point in response to patient movement. Rotation of the counterweight and drive gear can generate mechanical energy that is converted into electrical energy to power the implantable restriction device. In one exemplary embodiment, the kinetic motion apparatus includes a piezoelectric device that converts internal muscle and/or organ movement within a patient into energy to power the implantable restriction device. The piezoelectric device can also convert digestive motion of the stomach against the gastric band into electrical energy to power the implantable restriction device. The method can include storing excess energy generated by the kinetic motion apparatus in a storage device.

### BRIEF DESCRIPTION OF THE DRAWINGS

The invention will be more fully understood from the following detailed description taken in conjunction with the accompanying drawings, in which:

FIG. 1 is a representation of a food intake restriction system implanted in a patient to form a restriction in the patient's stomach;

FIG. 2 is a representation of a light powering device for powering the food intake restriction system of FIG. 1;

FIG. 3 is representation of a thermoelectric powering device for powering the food intake restriction system of FIG. 1;

FIG. 4 is a representation of one embodiment of a kinetic motion powering device for powering the food intake restriction system of FIG. 1;

FIG. 5 is a representation of another embodiment of a kinetic motion powering device for powering the food intake restriction system of FIG. 1; and

FIG. 6 is a representation of still another embodiment of a kinetic motion powering device for powering the food intake restriction system of FIG. 1.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are nonlimiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

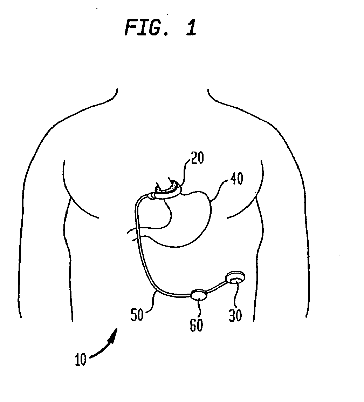

Various powering devices are provided for transferring energy from an external source through tissue to a communicating member implanted in a patient. The energy transferred to the communicating member can be used to provide power to an implantable restriction device that is implanted to form a restriction in a pathway within a patient. While the present invention disclosed herein can be used with a variety of implantable restriction devices known in the art, FIG. 1 illustrates one exemplary embodiment of a food intake restriction system 10. As shown, the system 10 generally includes an adjustable gastric band 20 that is configured to be positioned around the upper portion of a patient's stomach 40. In addition, the system 10 can include a communicating member capable of providing power to various devices configured to perform any number of tasks within the system 10, as will be described below.

The communicating member can be located anywhere in the system 10. For example, in one embodiment, the communicating member can be disposed within an injection port 30 shown in FIG. 1. The injection port 30 can be in fluid communication with the gastric band for allowing fluid to be introduced into and removed from the band to alter the amount of restriction provided by the band. Alternatively, or in addition, the communicating member can be disposed within a housing 60 that can house various components. In the illustrated embodiment, the system 10 includes both an injection port 30 and a housing 60. Both the injection port 30 and the housing 60 are coupled to the adjustable gastric band 20, e.g., via a catheter 50. A person skilled in the art will appreciate that the system need not include an injection port and/or housing, and that the communicating member can be positioned anywhere along the system 10.

In an exemplary embodiment, the communicating member can convert energy received from an external source to provide power to devices within the system 10 that measure and/or monitor various conditions of the system 10, that make adjustments to the gastric band 20 and/or other aspects of the system 10, and/or that measure/monitor various physiological parameters. Such devices can include, for example, sensors, pumps, bands and/or any other monitoring and/or adjustment devices having circuitry which requires electrical power. The communicating member can be configured to repeatedly receive energy from an external source, convert the energy to electrical power, and store the power in a capacitor, battery or other power storage device known in the art for later use by the device(s) within the system 10. Alternatively, the communicating member can be configured to transfer the converted power directly to the device(s) as needed. In addition, the communicating member can be configured to transmit and receive data to and from an external source. For example, the communicating member can receive command signals from an external source related to powering the system 10. The communicating member can also transmit various anatomical measurements taken within a patient's body to an external device or reader, as well as to transmit information regarding the charge status of the system 10.

The communicating member can take any form known in the art, and various embodiments of the communicating member are provided in detail below. In certain exemplary embodiments, the communicating member can take the form of a sensor capable of receiving energy from an external source for measuring and monitoring various parameters of the system 10; an antenna such as a dipole antenna, a monopole antenna with appropriate counterpoise, or an inductive coil capable of receiving energy through tissue; and/or any other devices known in the art which are capable of aiding in the powering, measuring, monitoring, and/or adjusting of the system 10 and/or other physiological parameters associated with the system 10.

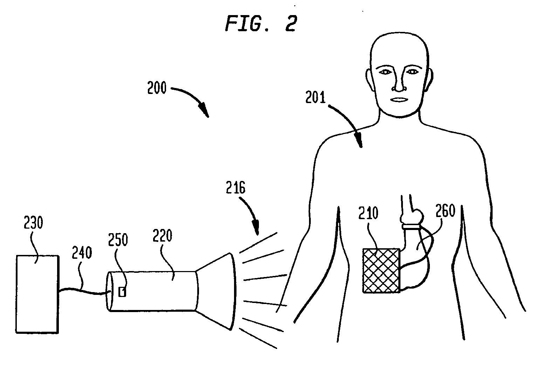

In one exemplary embodiment shown in FIG. 2, the communicating member is in the form of a photovoltaic cell array or solar cell 210 adapted to receive light waves 216 from an external apparatus 200. The external energy apparatus 200 can include a light source that generates the light waves 216. The light source can be configured in many ways known in the art, but in the illustrated embodiment, it is in the form of a hand-held external device 220 that is electrically connected to a power source 230, such as an electrical outlet or a battery, via an electric cable 240. The external device 220 can also include a switch 250 that enables a user to turn the external device 220 on or off as needed. When the external device 220 is in the "on" position, it can be configured to generate light waves 216 in the infrared range of about 0.70 µm to 1,000 µm. Alternatively or in addition, the external device 220 can be configured to generate light waves 216 in the visible range of about 400 nm to 750 nm or light waves 216 in the ultraviolet range of about 250 nm to 400 nm. Although not shown in FIG. 2, the external device 220 can also include a gauge effective to indicate whether light waves 216 are being communicated between the external device 220 and the solar cell 210 that are effective to power and/or charge the implantable restriction device. The indication given by the gauge can take the form of any notification means known in the art, including a light, such as an LED, an audible noise, and/or a vibration. Alternatively, a silicon nanowire can convert light energy into electric energy on the scale to power low power sensor devices.

In an exemplary embodiment, in use the solar cell 210 can be implanted beneath a tissue surface, e.g., in a patient's abdomen or fascia layer. A user can position the external device 220 in proximity to the implanted solar cell 210 and direct the light waves 216 towards a surface of the solar cell 210 implanted near a surface of a patient's skin. The solar cell 210 can receive and absorb the light waves 216, convert the light waves 216 into electrical power using methods well known in the art, and store them in a device, for example, a capacitor or battery, for later use by the implantable restriction device. Alternatively, the solar cell 210 can immediately transfer the energy via a cable 260 and/or via a wireless transfer to power other devices within the implantable restriction device for monitoring and/or adjusting the gastric band or performing other tasks as described above.

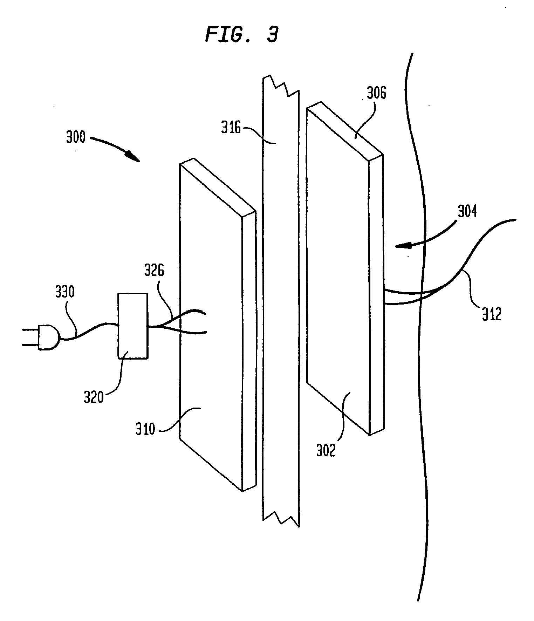

In another embodiment shown in FIG. 3, the communicating member can be in the form of a thermoelectric generator 306, such as a Peltier device, configured to use a temperature differential to generate electricity. The external device can include a thermoelectric powering device 300 adapted to power an implantable restriction device implanted within a patient. In an exemplary embodiment, the generator 306 can be implanted under a patient's skin and a temperature differential can be created across the generator 306 by providing an external temperature source which is different than body temperature. As shown, the generator 306 includes a first side 302, which faces outward from the patient's body and is positioned just under the skin. The generator 306 also includes a second side 304 which faces towards an interior of the patient's body. The generator 306 includes electrical leads 312 which can be connected to a storage device, such as a capacitor or battery, or directly to the devices within the implantable restriction device. A means for monitoring the charge level of the storage device may also be included.

As shown in FIG. 3, the thermoelectric powering device includes a temperature source 310. A person skilled in the art will appreciate that the temperature source 310 can be any device or element which is capable of producing a temperature that is different than the temperature associated with the second side 304 of the generator 306. For example, if the temperature of the second side 304 of the generator 306 is at an anatomical reference temperature such as a human body temperature, then the temperature source 310 can be a piece of ice which is at a temperature cooler than the anatomical reference temperature. Alternatively, the first side 302 of the generator 306 can be placed in contact with a large blood vessel within the body, since the body uses the blood stream to convey heat to and from the body. A natural temperature gradient exists in the body between the blood vessel and the body, and therefore between the first side 302 and the second side 304, which can be used to generate power.

In an exemplary embodiment, in use, when a patient or physician places the temperature source 310, e.g. ice, against a tissue surface 316, in proximity to the first side 302 of the implanted generator 306, a temperature differential is created across the generator 306, thereby causing it to generate electricity. A patient and/or physician can place the temperature source 310 against an area of the patient's skin that covers the first side 302 of the implanted generator 306. The temperature source 310 will change the temperature of the first side 302 of the generator so that there is a difference in temperature between the first side 302 and the second side 304 effective to generate electricity. In another example, the temperature source 310 can be a second Peltier device used as a thermoelectric cooler so that one side of the device is much cooler than the temperature of the second side 304 of the implanted generator 306. The thermoelectric cooler can then be placed adjacent to the tissue surface 316 in proximity to the first side 302 of the implanted generator 306, thereby creating a temperature differential across the generator 306 to produce electricity. Alternatively, the temperature source 310 can be eddy-current heating of a conductive component connected to or within the implantable restrictive device. The eddy current may be generated by an inductive coupled external alternating power source. Heating may be controlled for example by the mass of the conductive component, the size and shape of the component, magnetic permeability of the conductive component, resistivity of the conductive component, external power coupling frequency or the external power output level, etc. In one exemplary embodiment, the heat source could be a heating pad placed on or near the tissue surface. The electricity which is generated can then be used by devices within the implantable restriction device as needed.

The temperature source 310 can alternatively be connected to or disposed within an external device 320. The external device 320 can include a gauge that indicates whether a temperature differential exists between the temperature source 310 and the generator 306 that is effective to charge and/or power the implantable restriction device. The indication given by the gauge can take the form of any notification means known in the art, including a light, such as an LED, an audible noise, and/or a vibration. If the temperature source 310 is ice or another temperature element which doesn't require electrical power, an external device 320 may not be required for the purpose of providing power. If the temperature source 310 is a thermoelectric cooler or other electrically powered temperature source as illustrated in FIG. 3, then the external device 320 can provide power to the temperature source 310 via electrical leads 326. The external source 320 can contain batteries or other power source, or can be connected to a wall power source via cable 330.

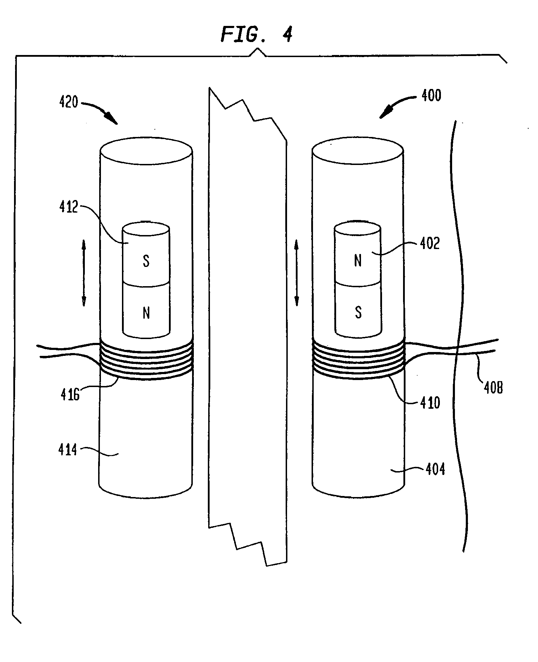

FIG. 4 shows another embodiment of a communicating member in the form of a kinetic motion apparatus 400 adapted to provide power to the implantable restriction device. In one exemplary embodiment, as shown, the kinetic motion apparatus 400 includes a housing having a magnet 402 disposed therein. The housing can be of any shape and made of any material known in the art, but in the illustrated embodiment, the housing is in the form of a glass tube or cylinder 404 having a metal or copper wire 410 wrapped tightly in a coil around an exterior surface of the cylinder 404. In this configuration, the kinetic motion apparatus 400 can generate electricity in the copper wire 406 by movement of the magnet 402 contained within the cylinder 404. Movement of the magnet 402 within the cylinder 404 will effectively cause the copper wire 410 to be moved through a magnetic field, thereby causing electricity to be generated, as will be appreciated by those skilled in the art. Electrical leads 408 coupled to the copper wire 410 are provided to carry the electricity generated by the kinetic motion apparatus 406 to a storage device or directly to devices within the implantable restriction device as needed. A means for monitoring the charge level of the storage device may also be included.

While many configurations are possible, in one exemplary embodiment, the kinetic motion apparatus 400 can be implanted within a patient's body such that physical movement of the body is effective to move the magnet 402 within the cylinder 404. For example, a patient can perform any movement, such as walking, running, jumping, shaking, etc., and this will cause the magnet 402 to move laterally, rotationally, or any combination thereof, within the cylinder 404 to generate electricity within the copper wire 406. In another example, the kinetic motion apparatus 400 may be implanted within a patient's body such that more subtle, but predictable physical movements within the body are effective in moving the magnet 402 within the cylinder 404. Examples of internal movements within the patient that may be harnessed include, but are not limited to, motions related to respiration (e.g., motions of the diaphragm), digestion (e.g., peristaltic waves through any portion of the gastrointestinal tract), and/or oscillatory motions within the circulatory system (e.g., pulsatile flow in the arterial system, motion of the heart, etc.).

Alternatively, or in addition, the kinetic motion apparatus 400 can include an external driver. In the embodiment shown in FIG. 4, the external driver is composed of the same elements as the kinetic motion apparatus 400, namely, a housing 414, a magnet 412, and a copper wire 416 to form an external electromagnet 420. The external electromagnet 420 can be manually driven by supplying the copper wire 416 with electricity to cause the magnet 412 to oscillate. As the magnet 412 oscillates, sympathetic oscillations are induced in the magnet 402 disposed within the kinetic motion apparatus 400, thereby causing electricity to be generated to supply power to the implantable restriction device. A person skilled in the art will appreciate that any driver or vibration element, internal or external, which is effective to produce oscillations, vibrations, or other motions in the magnet 402 within the kinetic motion apparatus 400, can be used to generate power. One additional alternative may include the conversion of oscillatory gradients in pressure created by natural and regularly occurring events such as respiration into fluid flows that induce oscillatory translational and/or rotational motions of the magnet 402. Moreover, the kinetic motion apparatus 400 can have a variety of other configurations in which energy is generated from motion or pressure gradients caused by these motions.

Although not shown in FIG. 4, an external device can also be provided to be in communication with the external driver and it can provide power to the external driver taken from a battery or other power source. The external driver can also include a gauge that indicates a charge status of the communicating member and/or whether there is proper alignment between an external driver and the kinetic motion apparatus 400. For example, the gauge can indicate whether circuitry and/or devices within the implantable restriction device need to be charged by the kinetic motion apparatus 400, or whether they are fully charged. Alternatively or in addition, the gauge can indicate proper alignment of an external driver that is attempting to generate sympathetic oscillations within the kinetic motion apparatus 400. The indication given by the gauge can take the form of any notification means known in the art, including a light, such as an LED, an audible noise, and/or a vibration.

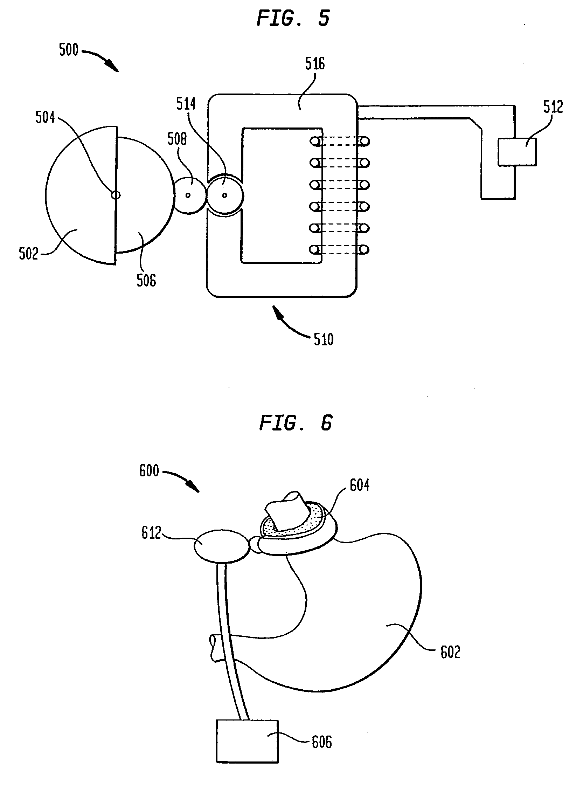

In another exemplary embodiment, a kinetic motion apparatus is provided that is operable to convert motion into energy to power the implantable restriction device. In one embodiment shown in FIG. 5, a kinetic motion apparatus 500 is provided and can include a counterweight 502 coupled to a shaft 504 such that the counterweight 502 can freely pivot about the shaft 504 in response to motion and movement of the patient. The counterweight 502 and the shaft 504 can be formed from any biocompatible material known in the art, including stainless steel, titanium, cobalt chrome, and any number of polymer plastics. A drive gear 506 can be nested within a hollow portion of the counterweight 502, and in one embodiment, it can be directly coupled to the counterweight 502 such the drive gear 506 moves in response to movement of the counterweight 502. The drive gear 506 can also be coupled to a drive train of an electric generator 510. As the drive gear 506 moves in response to the counterweight 502, it rotates a pinion gear 508 which in turn rotates the rotor 514 to a high velocity. This rotation then induces electric current through the stator 516 thereby charging the capacitor 512. The electric generator 510 thus converts mechanical energy from movement of the counterweight 503 into electrical energy.

The electrical energy produced by the generator 510 can be used to directly power the implantable restriction device or it can be stored within an accumulation element 512 for later use. In an exemplary embodiment, the accumulation element 512 can be a capacitor that contains lithium ion which provides an efficient conducting surface that may store energy longer than those capacitors typically made from other substrates. In another embodiment, the accumulation element 512 can be a high density ultracapacitor. A person skilled in the art will appreciate that any combination of gearing can be used to couple a patient's movement to the generator and any type of accumulation element 512 can be used to store charge.

In another embodiment shown in FIG. 6, a kinetic motion apparatus 600 is provided such that motion of a stomach 602 pushing against fluid in the gastric band 604 is converted into energy to supply power to a rechargeable battery or an accumulation element 606 that stores charge. As food passes through the band 604, pressure will increase and decrease in the gastric band 604. This vibration energy can be harvested by a variety of different methods known in the art such as electromagnetic, electrostatic, or piezoelectric conversion. In piezoelectric (piezo) methods, a bimorph based on piezoelectric materials vibrates, creating a charge that generates a voltage with amplitude proportional to the size and shape of the piezoelectric material, periodicity, and amount of force. Thus, the kinetic motion apparatus 600 can include a piezoelectric transducer element 612 attached to the gastric band 604 that can produce power proportional to the displacement and periodicity of band movement. This energy can then be stored in the accumulation element 606 until needed by the implantable restriction device. A person skilled in the art will appreciate that similar use can be made of electro-active polymer elements attached to the gastric band.

The internal devices disclosed herein are designed to be single use devices. The external devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application. The implantable devices disclosed herein are designed for single patient use.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.
The following is a non-exhaustive list of embodiments of the invention that may be claimed in this application or in subsequently filed divisional applications:
1. A system for forming a restriction in a patient, comprising:
   an implantable restriction device adapted to form a restriction in a pathway, the implantable restriction device including a communicating member adapted to utilize a temperature differential to power the implantable restriction device.
2. The system of embodiment 1, further comprising an external energy transfer apparatus having a temperature source operable to create a temperature differential across the communicating member to power the implantable restriction device.
3. The system of embodiment 1, wherein the communicating member is configured to utilize a temperature differential between the temperature source and an anatomical reference temperature to produce energy to power the implantable restriction device.
4. The system of embodiment 2, wherein the temperature source comprises a cold liquid or cold solid.
5. The system of embodiment 2, wherein the temperature source comprises a thermoelectric cooler.
6. The system of embodiment 2, wherein the temperature source comprises a heat source.
7. The system of embodiment 2, wherein the external energy transfer apparatus includes a gauge effective to indicate whether a temperature differential between the temperature source and the communicating member is effective to power the implantable restriction device.
8. The system of embodiment 1, wherein the communicating member comprises a thermogenerator.
9. The system of embodiment 1, wherein the implantable restriction device comprises a gastric band and a housing in communication with the gastric band.
10. The system of embodiment 9, wherein the communicating member is disposed in the housing.
11. The system of embodiment 1, wherein the communicating member is configured to receive and transmit data.
12. A method for providing power to an implantable restriction device, comprising:
   placing a temperature source on or near a tissue surface adjacent to a communicating member disposed within an implantable restriction device implanted in a patient, the communicating member utilizing a temperature differential to power the implantable restriction device.
13. The method of embodiment 12, wherein the temperature source is on an external device, and the external device receives data from the communicating member.
14. The method of embodiment 12, wherein the communicating member comprises a thermogenerator that powers the implantable restriction device.
15. The method of embodiment 14, wherein the temperature source is ice placed on or near a tissue surface adjacent to the thermogenerator creating a temperature differential with an anatomical reference temperature across the thermogenerator to produce electrical current to power the implantable restriction device.
16. The method of embodiment 14, wherein the temperature source is a thermoelectric cooler placed on or near a tissue surface adjacent to the thermogenerator creating a temperature differential with an anatomical reference temperature across the thermogenerator to produce electrical current to power the implantable restriction device.
17. The method of embodiment 14, wherein the temperature source is a heating source placed on or near a tissue surface adjacent to the thermogenerator creating a temperature differential with an anatomical reference temperature across the thermogenerator to produce electrical current to power the implantable restriction device.
18. The method of embodiment 14, wherein the temperature source is a blood vessel creating a temperature differential with an anatomical reference temperature across the thermogenerator to produce electrical current to power the implantable restriction device.
19. The method of embodiment 13, wherein the external device includes a gauge that indicates whether a temperature differential exists between the temperature source and the communicating member effective to power the implantable restriction device.
20. The method of embodiment 13, wherein the data includes at least one measurement of pressure of fluid within the implantable restriction device.
21. The method of embodiment 12, wherein the implantable restriction device comprises a gastric band disposed around a stomach to form a restriction, and a housing in communication with the gastric band.
22. The method of embodiment 12, wherein the communicating member measures a pressure and temperature of fluid in the implantable restriction device.

## Claims

1. A system for forming a restriction in a patient, comprising:
an implantable restriction device adapted to form a restriction in a pathway, the implantable restriction device including a communicating member adapted to utilize a temperature differential to power the implantable restriction device.

2. A method for providing power to an implantable restriction device, comprising:
placing a temperature source on or near a tissue surface adjacent to a communicating member disposed within an implantable restriction device implanted in a patient, the communicating member utilizing a temperature differential to power the implantable restriction device.

3. The system of claim 1, further comprising an external energy transfer apparatus having a temperature source operable to create a temperature differential across the communicating member to power the implantable restriction device.

4. The system of claim 1, wherein the communicating member is configured to utilize a temperature differential between the temperature source and an anatomical reference temperature to produce energy to power the implantable restriction device.

5. The system of claim 3, wherein the temperature source comprises a cold liquid, a cold solid, a thermoelectric cooler, or a heat source.

6. The system of claim 3 or the method of claim 2, wherein the external energy transfer apparatus of the system, or the external device of the method, includes a gauge effective to indicate whether a temperature differential between the temperature source and the communicating member is effective to power the implantable restriction device.

7. The system of claim 1, wherein the communicating member comprises a thermogenerator.

8. The system of claim 1 or the method of claim 2, wherein the implantable restriction device comprises a gastric band and a housing in communication with the gastric band.

9. The system of claim 8, wherein the communicating member is disposed in the housing.

10. The system of claim 1, wherein the communicating member is configured to receive and transmit data.

11. The method of claim 2, wherein the temperature source is on an external device, and the external device receives data from the communicating member.

12. The method of claim 2, wherein the communicating member comprises a thermogenerator that powers the implantable restriction device.

13. The method of claim 12, wherein the temperature source is:
a blood vessel; or
ice, a heating source or a thermoelectric cooler placed on or near a tissue surface adjacent to the thermogenerator;
creating a temperature differential with an anatomical reference temperature across the thermogenerator to produce electrical current to power the implantable restriction device.

14. The method of claim 11, wherein the data includes at least one measurement of pressure of fluid within the implantable restriction device.

15. The method of claim 2, wherein the communicating member measures a pressure and temperature of fluid in the implantable restriction device.
